# EUROPEAN PATENT SPECIFICATION

(11) **EP 3 482 761 B1**
(45) Date of publication and mention of the grant of the patent: **24.04.2024**
(21) Application number: 17830533.0
(22) Date of filing: 06.07.2017
(51) Int. Cl.: A61K 31/352, A61K 8/49, A61P 17/06, A61P 17/00, C07D 311/24, A61K 36/47, A61K 31/164, A61P 17/04

(54) **COMPOSITIONS COMPRISING CROMOGLICIC ACID FOR THE TREATMENT OF DERMATITIS**
ZUSAMMENSETZUNGEN MIT CROMOGLICINSÄURE ZUR BEHANDLUNG VON DERMATITIS
COMPOSITIONS D'ACIDE CROMOGLICIQUE POUR LE TRAITEMENT DE LA DERMATITE

(30) Priority: 07.07.2016 ES 201630928
(43) Date of publication of application: 15.05.2019
(73) Proprietor: Nutra Essential OTC, S.L., 28108 Alcobendas, Madrid (ES)
(72) Inventor: PALACIOS PELÁEZ, Ricardo, E-28918 Leganés (Madrid) (ES); ALCOVER DÍAZ, Javier, Madrid (ES); RODRÍGUEZ GIL, David, Madrid (ES); PINEDA DE LA LOSA, Fernando, Madrid (ES); TIANA FERRER, Concepción, E-28108 Alcobendas (Madrid) (ES); FERNÁNDEZ LORENZANA, Laura, E-28108 Alcobendas (Madrid) (ES); SÁNCHEZ GARCÍA, José Ángel, E-28108 Alcobendas (Madrid) (ES); VICARIO DE LA TORRE, Marta, E-28108 Alcobendas (Madrid) (ES)
(74) Representative: ABG Intellectual Property Law, S.L.
(86) International application number: PCT/ES2017/070490
(87) International publication number: WO 2018/015593

(56) References cited:
- WO-A1-99/27926
- WO-A1-2008/148572
- WO-A1-2011/012737
- WO-A2-2008/148573
- WILLIAM C. GORDON ET AL: "A Nonsteroidal Novel Formulation Targeting Inflammatory and Pruritus-Related Mediators Modulates Experimental Allergic Contact Dermatitis", DERMATOLOGY AND THERAPY, vol. 8, no. 1, 16 February 2018 (2018-02-16), pages 111-126, XP55663884, ISSN: 2193-8210, DOI: 10.1007/s13555-018-0223-8
- EYAL, Z.: "Topical Use of Sodium Cromoglicate (Cromolyn Sodium) To Treat Atopic Dermatitis and Other Skin Allergies", International Journal of Pharmaceutical Compounding, vol. 16, no. 5, September 2012 (2012-09), pages 386-393, XP009514916, ISSN: 1516-5140
- EBNER, F. et al.: "Topical Use of Dexpanthenol in Skin Disorders", American Journal of Clinical Dermatology, vol. 3, no. 6 2002, pages 427-433, XP009181863, ISSN: 1179-1888 Retrieved from the Internet: URL:https://doi.org/10.2165/00128071-20020 3060-00005
- JONES, K et al.: "Review of Sangre de Drago (Croton lechleri)-A South American Tree Sap in the Treatment of Diarrhea, Inflammation, Insect Bites, Viral Infections, and Wounds: Traditional Uses to Clinical Research", The Journal of Alternative and Complementary Medicine, vol. 9, no. 6, 2003, pages 877-896, XP009141651, ISSN: 1557-7708

## Description

### Field of the Invention

The present invention relates to the dermatological use of new compositions comprising cromoglicic acid or a salt or solvate thereof.

### Background of the Invention

Dermatitis is an acute or chronic inflammatory reaction of the skin that may be marked by varying degrees of severity. Dermatitis is a disease involving the participation of constitutional factors, for example higher immune sensitivity and genetic alterations, as well as a number of exposure factors which induce or help to maintain and exacerbate symptoms.

In general terms, at the onset of dermatitis edematous erythema can be observed, which is followed by erythematous lesions having papules and serous papules, followed by the formation of vesicles, pustules, erosions, scabs, and desquamations. Where dermatitis is acute, these states can be reverted over time in a natural manner. However, this entire process is usually recurrent. When dermatitis becomes chronic, thickening of the skin, lichenification, and pigmentations, which are often associated with itching, can additionally be observed.

Dermatitis is usually divided into different types including, among others, atopic dermatitis, contact dermatitis, seborrheic dermatitis, eczemas, psoriasis, and the like.

Current common treatments for dermatitis involve identifying and preventing factors that may induce or exacerbate inflammation, as well as good skin care, almost always combined with the use of drugs suitable for treating the symptoms of the disease. Among said drugs, steroidal formulations are commonly used. However, despite their proven clinical efficacy, steroid-based formulations, such as topical corticosteroid preparations, for example, cause a number of side effects, such as the excessive thinning of the skin, atrophy, the so-called "moon face" caused by the deposition of fat in the face, reddening of the skin, hirsutism, and stretch marks, among others. Furthermore, when the patient is exposed to a long-term use of steroids, the body usually develops resistance to those drugs, and there may even be cases where the symptoms of dermatitis come back in a more aggressive form.

Different types of non-steroidal therapies against dermatitis are known and used today. In particular, use of cromoglicic acid or derivatives thereof is suggested in the state of the art.

Cromoglicic acid is a mast cell stabilizer and is commonly sold in its sodium salt (sodium cromoglycate) form. The most common uses of this drug are for the treatment of asthma and conjunctivitis.

Document WO 99/60997 A1 describes formulations comprising sodium cromoglycate including an amphoteric surfactant and an alkoxylated cetyl alcohol as essential ingredients for the treatment of atopic dermatitis.

Document EP 0084190 A2 describes a composition comprising cromoglicic acid, or salts thereof, as well as a hydrophilic macromolecular material. The composition is useful in the treatment of skin wounds and lesions generating secretions, such as those that are formed along with dermatitis.

Document EP 1040826 A1 discloses cromoglicic acid esters which are useful in the treatment of cases of dermatitis in which an allergic process takes place.

EYAL, Z. et al (Int. J. Pharm. Compd., vol. 16, no. 5, 2012, 386-393) discloses the topical use of sodium cromoglicate to treat atopic dermatitis and other skin allergies.

EBNER, F. et al. (Am. J. Clin. Dermatol., vol. 3, no. 6, 2002, 427-433) describes the topical use of dexpanthenol in skin disorders.

JONES, K et al. (J. Altern. Complement. Med., vol. 9, no. 6, 2003, 877-896) describes the use of Croton lechleri in the treatment of diarrhea, inflammation, insect bites, viral infections, and wounds.

Despite the existence of the different cromoglicic acid-based formulations which are suggested in the state of the art for treating dermatitis, there is a continued need to develop new alternative dermatological compositions which enrich the available treatment arsenal.

### Summary of the Invention

The authors of the present invention have now discovered a combination of ingredients comprising cromoglicic acid or a salt or a solvate thereof which is suitable for treating dermatitis. It has furthermore been discovered that the ingredients of the combination unexpectedly act in a synergistic manner, thereby offering a particularly effective and/or safe treatment against dermatitis.

Therefore, in a first aspect the invention relates to a combination comprising:
a) cromoglicic acid or a salt or solvate thereof;
b) *Croton lechleri* resin; and
c) Panthenol
wherein the proportion by weight between the weight of the cromoglicic acid or the salt or solvate thereof, and the weight of panthenol, is 2.5:2.000 or greater.

In a second aspect, the invention relates to a pharmaceutical composition comprising the combination of the first inventive aspect and a pharmaceutically acceptable vehicle.

Another aspect of the present invention relates to the combination of the first aspect of the invention or to the composition of the second aspect of the invention for use as a medicament, particularly for use in the treatment or prophylaxis of atopic dermatitis.

Still another object of the present invention is the cosmetic (not therapeutic) use of the combination of the first aspect of the invention or of the composition of the second aspect of the invention in treating the skin to alleviate the effects derived from atopic dermatitis.

### Brief Description of the Drawings

Figure 1 shows the changes in ear thickness during the sensitization phase and during the subsequent treatment phase of the assay described in Example 2. Each entry corresponds with a group of mice and represents the mean ear thickness recorded based on the ear thickness of all the ears collected in said group.
Figure 2 shows the changes in ear thickness during the sensitization phase and during the subsequent treatment phase of the assay described in Example 3. Each entry corresponds with a group of mice and represents the mean ear thickness recorded based on the ear thickness of all the ears collected in said group.
Figure 3 shows the changes in PAR2 levels in mouse ear tissue after treatment with the compositions of the present invention.

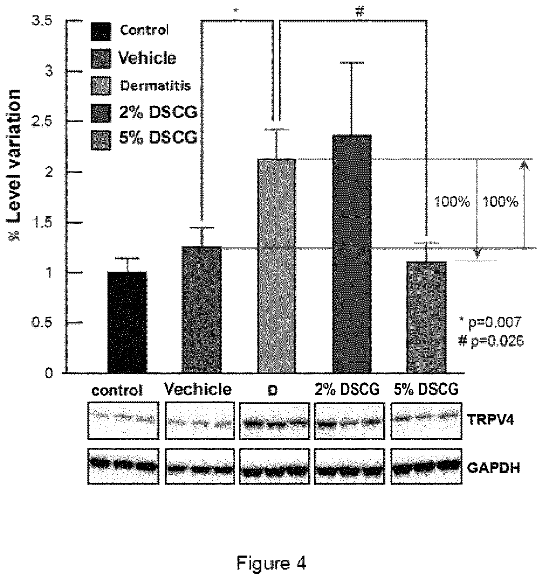

Figure 4 shows the changes in TRVP4 levels in mouse ear tissue after treatment with the compositions of the present invention.

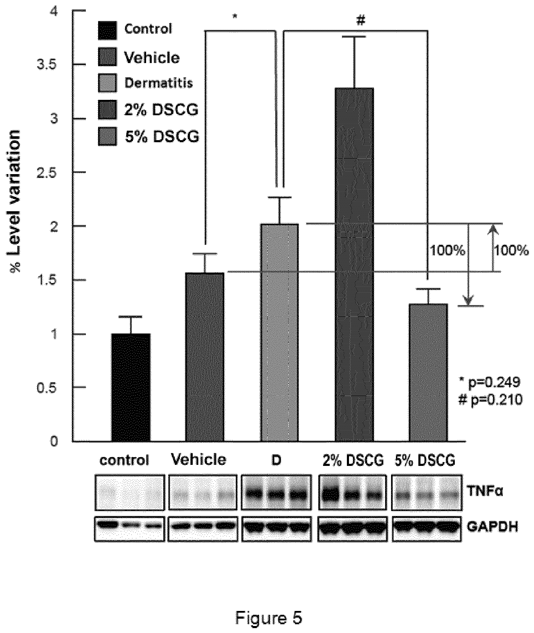

Figure 5 shows the changes in TNF-alpha levels in mouse ear tissue after treatment with the compositions of the present invention.

### Detailed Description of the Invention

An object of the present invention is a combination comprising:
a) cromoglicic acid or a salt or solvate thereof;
b) *Croton lechleri* resin; and
c) Panthenol
wherein the proportion by weight between the weight of the cromoglicic acid or the salt or solvate thereof, and the weight of panthenol, is 2.5:2.000 or greater.

### Cromoglicic acid

The combination of the present invention comprises cromoglicic acid or a salt or solvate thereof. Cromoglicic acid (IUPAC name: 5,5'-(2-hydroxypropane-1,3-diyl)bis(oxy)bis(4-oxo-4*H*-chromene-2-carboxylic acid) is a mast cell stabilizer typically known for its usefulness in the treatment of asthma and conjunctivitis. The formula of the compound is as follows:

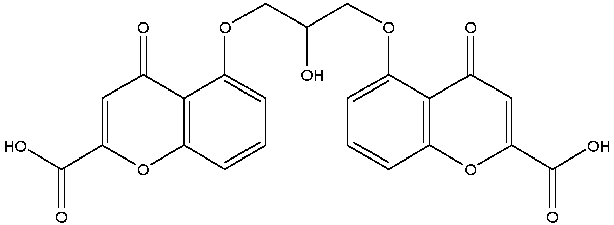

Cromoglicic acid can be obtained from commercial sources or be prepared by chemical synthesis methods described in the state of the art or variations thereof which fall under the common general knowledge of one skilled in the art. For example, cromoglicic acid can be prepared as described in patent application EP 1040826 A1.

In the context of the present invention, the salt of cromoglicic acid, is a pharmaceutically acceptable salt. A pharmaceutically acceptable salt is understood to mean any salt that is physiologically tolerated when used in a suitable manner for a treatment that is applied or used, particularly in human beings and/or mammals. Preferably, the pharmaceutically acceptable salt is a dermatologically acceptable salt, i.e., it is a salt that is suitable for being applied on the skin which does not show any problems relating to toxicity, incompatibility, irritation, allergic response, or the like.

Preferably, the salt is one in which the cromoglicic acid forms the anion, preferably and where appropriate, by the deprotonation of at least one carboxylic and/or hydroxyl group, and the counter cation is an alkaline or alkaline-earth metal, or an ammonium cation. Preferably, the counter cation is an alkaline metal. More preferably, the counter cation is sodium or potassium. Even more preferably, the counter cation is sodium.

Preferably, the salt is a cromoglicic acid salt in which one or both of the carboxylic groups and/or the secondary hydroxyl group are in a deprotonated state, and the counter cation is an alkaline or alkaline-earth metal, or an ammonium cation. Preferably, the counter cation is an alkaline metal. More preferably, the counter cation is sodium or potassium. Even more preferably, the counter cation is sodium.

More preferably, the salt is a cromoglicic acid salt in which the carboxylic groups are in a deprotonated state, and the counter cation is an alkaline or alkaline-earth metal, or an ammonium cation. Preferably, the counter cation is an alkaline metal. More preferably, the counter cation is sodium or potassium. Even more preferably, the counter cation is sodium.

In a particularly preferred embodiment, the cromoglicic acid is sodium cromoglycate, which can be monosodium or disodium cromoglycate, i.e., the cromoglicic acid salt in which, respectively, one or both carboxylic groups are in a deprotonated state, and the counter cation (of each deprotonated carboxylic group) is sodium. Preferably, the cromoglicic acid is disodium cromoglycate (DSCG).

The salts mentioned above can be obtained from commercial sources or be prepared according to methods that are well-known in the field of the invention. For example, the salts in which the counter cation of the carboxylic groups is an alkaline or alkaline-earth metal can be prepared by reacting cromoglicic acid with hydroxides or alkoxides of alkaline or alkaline-earth metals in a suitable solvent.

In the context of the present invention, the solvate of the cromoglicic acid, even more preferably of disodium cromoglycate, is any form in which said compound is bound by means of a noncovalent bond to another molecule (usually a polar solvent), particularly including hydrates and alcoholates, such as methanolate, for example. A preferred solvate is a hydrate.

### Croton lechleri resin

*Croton lechleri* is the scientific name of the tree referred to as *"sangre de drago"* (dragon's blood), which belongs to the *Euphorbiaceae* family. A resin is extracted from said tree, and the most active components of said resin are proanthocyanidins and the alkaloid taspine. Other components of the resin include lignans, polyunsaturated fatty acids, pigments, and flavonoids.

The properties and traditional applications of *C*. *lechleri* resin are described in the review paper by K. Jones, Review of Sangre de Drago (Croton lechleri) - A South American Tree Sap in the Treatment of Diarrhea, Inflammation, Insect Bites, Viral Infections, and Wounds: Traditional Uses to Clinical Research, J. Altern. Complem. Med., 2003, 9(6), 877-896.

*C. lechleri* resin can be found on the market, for example in the form of a hydroglycolic solution (water and propylene glycol), under the name Dragon's Blood, sold by the company Cobiosa, in which the resin content is comprised between 1% and 5% by weight.

Therefore, in an embodiment the *C*. *lechleri* resin comprised in the combination of the invention is a hydroglycolic solution, preferably based on water and propylene glycol. Preferably, this hydroglycolic solution comprises the resin in a content by weight of between 1% and 5%.

### Panthenol

Panthenol is a compound which belongs to the vitamin B group and is converted into pantothenic acid (vitamin B₅) in the skin, so it is also referred to as provitamin B₅.

Said compound is well known in the cosmetics industry as it has been used for years as a skin moisturizing agent.

Panthenol is a chemical compound which has a chiral carbon atom, so it exists in two enantiomeric forms, of which forms, only D-panthenol (dexpanthenol) is biologically active. Nevertheless, both enantiomers show moisturizing activity, and both D-panthenol and the racemic mixture including D-panthenol and L-panthenol can be used in cosmetic compositions.

In a preferred embodiment, in the combination of the invention the panthenol is selected from D-panthenol and the racemic mixture, and preferably D-panthenol is used.

### Combinations

In an embodiment, the proportion between *Croton lechleri* resin and panthenol is comprised between 1:500 and 1:2, expressed as weight:weight, preferably between 1:200 and 1:10, particularly between 1:100 and 1:20, in a particularly preferred manner between 1:50 and 1:30, and particularly between 1:50 and 1:40, more particularly between 1:45 and 1:44.

The combination of the weight of *Croton lechleri* resin and panthenol forms weight B. In a preferred embodiment, the proportion by weight between the weight of the cromoglicic acid (or a salt or solvate thereof), preferably disodium cromoglycate (or a solvate thereof), and weight B is 2.5:2.045 or greater (greater being understood to mean that the weight of cromoglicic acid is increased or weight B is decreased), 4.5:2.045 or greater, or 4.9:2.045 or greater. More specifically, the proportion is between 2.5:2.045 and 10:2.045, between 4.5:2.045 and 10:2.045, or between 4.9:2.045 and 10:2.045. More specifically, the proportion is between 2.5:2.045 and 5.5:2.045, between 4.5:2.045 and 5.5:2.045, or between 4.9:2.045 and 5.5:2.045. More specifically, the proportion is between 2.5:2.045 and 5.1:2.045, between 4.5:2.045 and 5.1:2.045, or between 4.9:2.045 and 5.1:2.045.

In the present invention, the proportion by weight between the cromoglicic acid (or a salt or solvate thereof), preferably disodium cromoglycate (or a solvate thereof), and the weight of panthenol is 2.5:2.000 or greater (greater being understood to mean that the weight of cromoglicic acid is increased or the weight of panthenol is reduced), 4.5:2.000 or greater, or 4.9:2.000 or greater. More specifically, the proportion is between 2.5:2.000 and 10:2.000, between 4.5:2.000 and 10:2.000, or between 4.9:2.000 and 10:2.000. More specifically, the proportion is between 2.5:2.000 and 5.5:2.000, between 4.5:2.000 and 5.5:2.000, or between 4.9:2.000 and 5.5:2.000. More specifically, the proportion is between 2.5:2.000 and 5.1:2.000, between 4.5:2.000 and 5.1:2.000, or between 4.9:2.000 and 5.1:2.000.

In a preferred embodiment, the proportion by weight between the weight of the cromoglicic acid (or a salt or solvate thereof), preferably disodium cromoglycate (or a solvate thereof), and the weight of the *Croton lechleri* resin is 2.5:0.045 or greater (greater being understood to mean that the weight of cromoglicic acid is increased or the weight of the *Croton lechleri* resin is reduced), 4.5:0.045 or greater; or of 4.9:0.045 or greater. More specifically, the proportion is between 2.5:0.045 and 10:0.045, between 4.5:0.045 and 10:0.045, or between 4.9:0.045 and 10:0.045. More specifically, the proportion is between 2.5:0.045 and 5.5:0.045, between 4.5:0.045 and 5.5:0.045, or between 4.9:0.045 and 5.5:0.045. More specifically, the proportion is between 2.5:0.045 and 5.1:0.045, between 4.5:0.045 and 5.1:0.045, or between 4.9:0.045 and 5.1:0.045.

In a particular embodiment, the combination of the invention consists essentially of:
a) cromoglicic acid (or a salt or solvate thereof), preferably disodium cromoglycate (or a solvate thereof);
b) *Croton lechleri* resin;
c) panthenol.

According to this embodiment, the combination cannot comprise additional ingredients which are active ingredients against dermatitis.

In a particular embodiment, the combination of the invention consists of:
a) cromoglicic acid (or a salt or solvate thereof), preferably disodium cromoglycate (or a solvate thereof);
b) *Croton lechleri* resin;
c) panthenol.

The combinations of the present invention can comprise additional ingredients.

In a particular embodiment, the combination of the invention comprises aloe vera gel. The aloe vera gel is obtained from the succulent plant *Aloe barbadensis* of the *Asphodelaceae* family. The leaves of said plant are made up of three layers: an outer leathery protective layer, under that a fibrous layer, and a gel core where it stores its water reserves and which is used to prepare dermatological and cosmetic products. Aloe vera gel extracted from the plant with a solid content of about 0.5% by weight, referred to as gel 1:1, can be found on the market. It can also be found in the form of a concentrated gel from which some of the water has been extracted. For example, aloe vera gel concentrate 2:1 has a solid content of about 1% by weight; aloe vera gel concentrate 10:1 has a solid content of about 5% by weight; aloe vera gel concentrate 40:1 has a solid content of about 20% by weight. Spray-dried products 100:1 and 200:1, which allow reconstituting the original gel when mixing together one part of said product and 99 parts of water or 1 part of said product and 199 parts of water, respectively, can also be found.

In the context of the invention, an aloe vera gel concentrate 10:1 is preferably used, such that the combination of 1 part of said gel with 9 parts of water yields the original gel of the plant.

In another particular embodiment, the combination of the invention comprises rosehip oil.

The rosehip is a plant which belongs to the *Rosaceae* family that includes three species: *Rosa moschata, Rosa canina,* and *Rosa rubiginosa.* It is a bush with thin, flexible branches with many thorns and it may grow to over 2 meters in height. Today it can be found all over many areas of the planet, particularly in regions with a temperate climate. The oil is extracted from the seeds its fruits contain.

In an embodiment, the present invention relates to a combination as previously defined, which additionally comprises:
d) aloe vera gel and/or rosehip oil.

In a particular embodiment, the combination of the invention consists essentially of:
a) cromoglicic acid (or a salt or solvate thereof), preferably disodium cromoglycate (or a solvate thereof);
b) *Croton lechleri* resin;
c) panthenol; and
d) aloe vera gel and/or rosehip oil.

According to this embodiment, the combination cannot comprise additional ingredients which are active ingredients against dermatitis.

In a particular embodiment, the combination of the invention consists of:
a) cromoglicic acid (or a salt or solvate thereof), preferably disodium cromoglycate (or a solvate thereof);
b) *Croton lechleri* resin;
c) panthenol; and
d) aloe vera gel and/or rosehip oil.

In a preferred embodiment, the proportion by weight between the weight of the cromoglicic acid (or a salt or solvate thereof), preferably disodium cromoglycate (or a solvate thereof), and the weight of the aloe vera gel (preferably concentrate 10:1) is 2.5:3.000 or greater (greater being understood to mean that the weight of cromoglicic acid is increased or the weight of the aloe vera gel is reduced), of 4.5:3.000 or greater, or 4.9:3.000 or greater. More specifically, the proportion is between 2.5:3.000 and 10:3.000, between 4.5:3.000 and 10:3.000, or between 4.9:3.000 and 10:3.000. More specifically, the proportion is between 2.5:3.000 and 5.5:3.000, between 4.5:3.000 and 5.5:3.000, or between 4.9:3.000 and 5.5:3.000. More specifically, the proportion is between 2.5:3.000 and 5.1:3.000, between 4.5:3.000 and 5.1:3.000, or between 4.9:3.000 and 5.1:3.000.

In a preferred embodiment, the proportion by weight between the weight of the cromoglicic acid (or a salt or solvate thereof), preferably disodium cromoglycate (or a solvate thereof), and the weight of the rosehip oil is 2.5:0.500 or greater (greater being understood to mean that the weight of cromoglicic acid is increased or the weight of the rosehip oil is reduced), of 4.5:0.500 or greater, or 4.9:0.500 or greater. More specifically, the proportion is between 2.5:0.500 and 10:0.500, between 4.5:0.500 and 10:0.500, or between 4.9:0.500 and 10:0.500. More specifically, the proportion is between 2.5:0.500 and 5.5:0.500, between 4.5:0.500 and 5.5:0.500, or between 4.9:0.500 and 5.5:0.500. More specifically, the proportion is between 2.5:0.500 and 5.1:0.500, between 4.5:0.500 and 5.1:0.500, or between 4.9:0.500 and 5.1:0.500.

### Compositions

Another object of the invention is a pharmaceutical composition comprising the combination of the invention and a pharmaceutically acceptable vehicle. Pharmaceutically acceptable vehicle is understood to mean a vehicle which is physiologically tolerated when used in a suitable manner for a treatment that is applied or used, particularly in human beings and/or mammals, i.e., a vehicle which does not show any problems relating to toxicity, incompatibility, irritation, allergic response, or the like, and any incompatibilities with the ingredients of the combination of the invention. Preferably, the pharmaceutical composition is a dermatological composition, and the pharmaceutically acceptable vehicle is a dermatologically acceptable vehicle. Dermatological composition is understood to mean a pharmaceutical composition suitable for treating and/or preventing skin conditions, and dermatologically acceptable vehicle is understood to mean a vehicle suitable for being applied on the skin which does not show any problems relating to toxicity, incompatibility, irritation, allergic response, or the like in the skin.

In a preferred embodiment, the compositions of the invention comprise:
- between 4.7% and 27% by weight of the combination of the invention; and
- between 95.3% and 73% by weight of the pharmaceutically acceptable vehicle;
where the percentages of the components are adjusted such that the balance is 100%.

Preferably, the weight of the combination of the invention with respect to the total weight of the composition is between 5 and 20%, particularly between 7 and 15%, particularly between 10 and 11%, and even more particularly between 10.5 and 10.6%.

In an embodiment, the weight of the cromoglicic acid (or a salt or solvate thereof), preferably disodium cromoglycate (or a solvate thereof), is between 0.01%, 0.1%, 1%, and 30% with respect to the total weight of the composition. In another embodiment, it is between 0.01%, 0.1%, 1%, and 20%. In another embodiment, it is between 0.01%, 0.1%, 1%, and 10%.

In a preferred embodiment, the weight of the cromoglicic acid (or a salt or solvate thereof), preferably disodium cromoglycate (or a solvate thereof), is 2.5%, 3%, 3.5%, 4%, or 4.5% or greater with respect to the total weight of the composition. In another preferred embodiment, the weight is between 2.5%, 3%, 3.5%, 4%, or 4.5%, and 10% with respect to the total weight of the composition. In another more particular embodiment, the weight is between 2.5%, 3%, 3.5%, 4%, or 4.5%, and 8%. In another more particular embodiment, the weight is between 2.5%, 3%, 3.5%, 4%, or 4.5%, and 7%. In another more particular embodiment, the weight is between 2.5%, 3%, 3.5%, 4%, or 4.5%, and 6%. In another more particular embodiment, the weight is between 2.5%, 3%, 3.5%, 4%, or 4.5%, and 5.5%. In another more particular embodiment, the weight is between 2.5%, 3%, 3.5%, 4%, or 4.5%, and 5.1%. In another more particular embodiment, the weight is between 2.5%, 3%, 3.5%, 4%, or 4.5%, and 5%. In an even more particular embodiment, the weight is between 4.5% and 5.5%. In yet a more particular embodiment, the weight is between 4.9% and 5.1%.

As previously indicated, the weight of the *Croton lechleri* resin plus the weight of the panthenol, preferably D-panthenol, is weight B. In an embodiment, weight B is 0.2-12% with respect to the total weight of the composition, preferably 1-9%, more preferably 1-3%, even more preferably 1.5-2.5%, yet more preferably 2.0-2.1%. For each of these weights, the ratio by weight between the *Croton lechleri* resin and the panthenol, preferably D-panthenol, is comprised between 1:500 and 1:2, expressed as weight:weight, preferably between 1:200 and 1:10, particularly between 1:100 and 1:20, in a particularly preferred manner between 1:50 and 1:30, and particularly between 1:50 and 1:40, more particularly between 1:45 and 1:44.

In another embodiment, the weight of the aloe vera gel, preferably aloe vera gel concentrate 10:1, ranges between 2% and 4%, preferably between 2.5% and 3.5%, particularly between 2.9% and 3.1% with respect to the total weight of the composition, or it is 3.0%.

In another embodiment, the weight of the rosehip oil ranges between 0.01% and 1%, preferably between 0.1% and 1%, particularly between 0.3% and 0.8%, and particularly between 0.4% and 0.6%, more particularly between 0.45% and 5.5%, in an especially particular manner between 0.49% and 0.51% with respect to the total weight of the composition, or it is 0.50%.

Preferably, the ingredients of the combination of the invention are dissolved, emulsified, dispersed, or suspended in the pharmaceutically acceptable vehicle. Said vehicle is selected from water, a non-aqueous water-miscible vehicle, for example ethanol, isopropanol, and a non-aqueous, non water-miscible vehicle, for example paraffin oil. Preferably, the compositions of the invention include water as a pharmaceutically acceptable vehicle.

In other embodiments, the compositions of the invention further comprise at least one additional dermatological or cosmetic active ingredient.

In a particular embodiment, the weight of all the additional dermatological and cosmetic active ingredients is between 5 and 40% with respect to the total weight of the composition.

More particularly, the composition of the invention comprises:
- between 4.7% and 27% by weight of the combination of the invention;
- between 5% and 40% by weight of additional dermatological and cosmetic active ingredients (i.e., the overall weight of additional active dermatological and cosmetic ingredients); and
- between 33% and 90.3% by weight of the pharmaceutically acceptable vehicle;
where the percentages of the components are adjusted such that the balance is 100%.

In more specific embodiments, the weights of the combination of the invention are as previously defined.

In a more specific embodiment, the weight of the additional active dermatological and cosmetic ingredients is comprised between 10% and 35%, particularly between 15% and 30%, particularly between 20% and 25% with respect to the total weight of the composition.

In an embodiment, the additional dermatological active ingredient is a steroid, more preferably a corticosteroid. In a more particular embodiment, the corticosteroid is selected from prednisolone, hydrocortisone butyrate, dexamethasone valerate, betamethasone dipropionate, clobetasol propionate, and clobetasone butyrate.

In another embodiment, said additional dermatological active ingredient is an antihistamine. In a more particular embodiment, the antihistamine is selected from diphenhydramine hydrochloride, mequitazine, promethazine hydrochloride, and chlorpheniramine maleate.

In another embodiment, said additional dermatological active ingredient is an antiallergic agent. In a more particular embodiment, the antiallergic agent is selected from tranilast, ketotifen fumarate, oxatomide, azelastine hydrochloride.

In another embodiment, said additional dermatological active ingredient is an antibacterial agent, preferably an antibacterial agent useful for treating acne. In a more particular embodiment, the antibacterial agent is clindamycin phosphate or tetracycline.

In an embodiment, said cosmetic ingredient is selected from at least: a humectant, an emulsifying agent, an emollient, a silicone, a chelator, a preservative, and a pH regulator.

Humectants are substances which moisten and soften the skin. In the context of the present invention, humectants can be selected from, among others, glycerin, propylene glycol, glycols, polyethylene glycols, and mixtures thereof.

In an embodiment, the humectant content is comprised between 5 and 10% by weight with respect to the total weight of the composition.

Emulsifying agents favor the formation of intimate mixtures of non-miscible liquids due to an alteration of the interfacial tension. In the context of the present invention, the emulsifying agents can be selected from, among others, polysorbates, sorbitan esters, fatty alcohols, ethoxylated fatty alcohols, fatty acids, and mixtures thereof, such as beeswax.

In an embodiment, the emulsifying agent content is comprised between 5 and 10% by weight with respect to the total weight of the composition.

Emollients contribute to optimizing the organoleptic and dermatological properties of the composition. In the context of the present invention, the emollients can be selected from, among others, alkanes and esters, such as glycerides, propylene glycol esters, alkyl esters, ethers, glycols, and mixtures thereof.

The total amount of emollients is comprised between 8 and 12% by weight with respect to the total weight of the composition.

In the formulations of the present invention, silicones are also used, and while said silicones could be identified as a type of emollient, in the context of the present invention they are presented separately from the emollients. In the context of the present invention, the silicones can be selected from, among others, poly(dialkylsiloxanes), poly(diarylsiloxanes), and poly(alkylarylsiloxanes), such as dimethicone, hexamethylcyclotrisiloxane, phenyldimethicone, cyclomethicone, hexamethylcyclotrisiloxane, polydimethylsiloxane, or poly(methylphenylsiloxane), for example.

The total amount of silicones is comprised between 1 and 3% by weight with respect to the total weight of the composition.

Chelating agents complex and neutralize metallic ions which may affect the stability and/or appearance of the composition. The chelating agents can be monodentate and multidentate. In the context of the present invention, the chelating agents can be selected from, among others, ethylenediaminetetraacetic acid (EDTA), nitriloacetic acid (NTA), hydroxyethylethylenediaminetriacetic acid (HEEDTA), diethylenetriaminepentaacetic acid (DTPA), diethanol glycine (DEG), ethanoldiglycine (EDG), citric acid, phosphoric acid and tartaric acid, or the salts thereof, and mixtures thereof.

The total amount of chelating agents is comprised between 0.01 and 1% by weight with respect to the total weight of the composition.

Preservatives stop or minimize deterioration of the components of the formulation caused by the presence of different types of microorganisms. In the context of the present invention, the preservatives can be selected from, among others, phenoxyethanol, tropolone, chlorphenesin, ethylhexylglycerin, isothiazolidone, diazolidinyl urea, imidazolidinyl urea, and parabens, and mixtures thereof.

The total amount of preservatives is comprised between 0.5 and 1.5% by weight with respect to the total weight of the composition.

pH regulators establish and/or maintain the pH of the composition at the desired value. In the context of the present invention, the pH regulators can be selected from, among others, citric acid, acetic acid, phosphoric acid, propionic acid, lactic acid, carbonic acid, ammonium/ammonia, sodium hydroxide, and mixtures thereof.

The total amount of pH regulators is comprised between 0.01 and 1% by weight with respect to the total weight of the composition.

The compositions of the present invention contain water and a lipophilic phase, and they are generally presented in the form of emulsions or dispersions, for example, of the oil-in-water (O/W) or water-in-oil (W/O) type, multiple emulsions (W/O/W), or PIT-type emulsions, as described in the Spanish patent ES 2169908 T3, or as microemulsions. They can also be presented in the form of a suspension. Preferably, they are in the form of an emulsion. Even more preferably, they are in the form of an oil-in-water (O/W) type emulsion.

The compositions of the present invention are presented in various dosage forms, preferably dermatological dosage forms, even more preferably dermatological forms for topical application, even more preferably dermatological forms for topical application on the skin. For example, the compositions are presented in the form of a cream, salve, ointment, balsam, lotion, milk, gel, foam, gelatin, etc.

When the compositions are prepared to have a low viscosity, they can also be applied by spraying, i.e., in these cases the compositions can be presented in spray form, for example as a spray or aerosol. In a preferred embodiment, the compositions are a spray emulsion.

The compositions can also be incorporated in sponges, tapes, patches, dressings, or bandages. In a particular embodiment, the compositions are presented in the form of a patch, preferably a transdermal patch.

Preferably, the compositions of the invention are in the form of a cream, lotion, milk, or spray emulsion. Even more preferably, they are in the form of a cream, lotion, or milk. Even more preferably, they are in the form of a cream.

### Method of preparation

The compositions of the invention can be prepared following conventional methods in the cosmetics/pharmaceutical industry for the preparation of lotions, milks, creams, and spray emulsions. The preparation of emulsions is described, for example, in Remington: The Science and Practice of Pharmacy, 20th edition, Lippincott Williams & Wilkins, Philadelphia, 2000 [ISBN: 0-683-306472] .

A method for preparing a composition of the invention can be, for example, as follows.

In a reactor equipped with stirring equipment, the hydrophilic components such as water, glycerin, or panthenol are weighed and melted at a temperature comprised between 70°C and 85°C, preferably between 75°C and 80°C. In another vessel, the lipophilic components and emulsifiers are weighed and heated at a temperature comprised between 70°C and 85°C, preferably between 75°C and 80°C. Then an emulsion is prepared from the lipophilic mass and the hydrophilic components with suitable stirring. Finally, the obtained emulsion is cooled to a temperature comprised between 35°C and 45°C, preferably 40°C (+/- 2°C).

At the same time as performing the method of emulsion, water is heated in a different vessel at a temperature between 35°C and 45°C, preferably 40°C (+/- 2°C), and then the cromoglicic acid or a salt thereof, even more preferably disodium cromoglycate, is slowly added until achieving complete dissolution thereof, all while maintaining the temperature between 35°C and 45°C, preferably 40°C (+/- 2°C).

The solution of the cromoglicic acid is subsequently added, under stirring and keeping the temperature between 35°C and 45°C, preferably 40°C (+/- 2°C), to the emulsion which has been prepared at the same time. The mixture is homogenized, and the temperature between 35°C and 45°C, preferably 40°C (+/- 2°C), is maintained.

Subsequently, the *Croton lechleri* resin is added to the homogenized mixture. In those cases where aloe vera gel and/or rosehip oil are used, the following are added to the cooled mixture one by one and preferably in the following order: i) the *Croton lechleri* resin; ii) aloe vera gel; iii) rosehip oil. In both cases, any other component that may be sensitive to high temperatures is also added. Subsequently, in all cases, the obtained mixture is homogenized so as to obtain the composition of the invention. During this addition and homogenization phase, a temperature between 35°C and 45°C, preferably 40°C (+/- 2°C), is also maintained.

Finally, it is checked that the viscosity and pH are those desired. If they are not, viscosity can be modified by techniques that are common in the field of the invention. On the other hand, where necessary, the pH of the composition can also be adjusted by techniques that are common in the field of the invention, for example by the adding citric acid, hydrochloric acid, or sodium hydroxide.

Once these are checked, and where necessary adjusted, the composition is ready for packaging.

### Usefulness

The combinations of the invention, or the compositions comprising a combination according to the present invention, are useful as a pharmaceutical composition, preferably a dermatological composition, for treating or preventing dermatitis.

It has further been observed that the combinations of the invention are capable of regulating different molecular markers associated with dermatitis. It has specifically been observed that the combinations of the invention induce synthesis of the neuroprotective docosanoids, Neuroprotectin D1 and the isomers thereof; they reduce the production of the pruritus markers PAR2 (protease activated receptor 2) and TRPV1 and 4 (transient receptor potential vanilloid 1 and 4); and they reduce the production of inflammation markers COX-2 (cyclooxygenase-2) and TNF-alpha (tumor necrosis factor alpha). Particularly, these biomolecular effects can be observed or are particularly pronounced when the proportion by weight between the weight of the cromoglicic acid (or a salt or solvate thereof), preferably disodium cromoglycate (or a solvate thereof), and weight B is preferably 2.5:2.045 or greater, more preferably 4.5:2.045 or greater; or when the weight of the cromoglicic acid (or a salt or solvate thereof), preferably disodium cromoglycate (or a solvate thereof), is preferably 2.5% or greater with respect to the total weight of the composition, preferably 4.5% or greater.

Therefore, in a preferred embodiment the present invention relates to the combinations of the present invention for use in the treatment or prevention of dermatitis through the modulation of PAR2, more specifically through the reduction of the expression/levels of PAR2. The correlation between the PAR2 marker and dermatitis is widely documented in the state of the art, for example in Zhu et al., Int Immunopharmacol, 2015, 28:507-512; in Zhu et al., Int Immunopharmacol 2009, 9:1332-1336; or in Carvalho et al., Exp Dermatol, 2010, 19:117-122. In that sense, in an embodiment the combinations of the present invention are useful in the treatment or prevention of the itching and/or inflammation associated with dermatitis, and in a more particular embodiment the combinations of the present invention are useful in the treatment or prevention of the itching and/or inflammation associated with dermatitis through the modulation of, more specifically the reduction of, the expression/levels of PAR2.

Likewise, in another preferred embodiment the present invention relates to the combinations of the present invention for use in the treatment or prevention of dermatitis through the modulation of TRPV4, more specifically through the reduction of the expression/levels of TRPV4. The correlation between the TRPV4 marker and dermatitis is widely documented in the state of the art, for example in White et al., Physiol Rev, 2016, 96:911-973; or in Poole et al., J Biol Chem, 2013, 22, 288:5790-5802. In that sense, in a more particular embodiment, the combinations of the present invention are useful in the treatment or prevention of the itching and/or inflammation associated with dermatitis through the modulation of, more specifically the reduction of the expression/levels of, TRPV4.

In a more particular embodiment, the combinations of the present invention are useful in the treatment or prevention of the itching and/or inflammation associated with dermatitis through the modulation of, more specifically the reduction of the expression/levels of, PAR2 and TRPV4.

Likewise, in another preferred embodiment, the present invention relates to the combinations of the present invention for use in the treatment or prevention of dermatitis through the modulation of TNF-alpha, more specifically through the reduction of the expression/levels of TNF-alpha. The correlation between the TNF-alpha marker and dermatitis is widely documented in the state of the art, for example in Dinarello, Chest, 2000, Aug 118(2):503-8; or in Danso et al. J Invest Dermatol, 2014, Jul 134(7):1941-50. In that sense, in a more particular embodiment the combinations of the present invention are useful in the treatment or prevention of the inflammation associated with dermatitis through the modulation of, more specifically the reduction of the expression/levels of, TNF-alpha.

In an embodiment, the itching or inflammation associated with dermatitis are, respectively, itching or inflammation in subjects suffering from dermatitis (treatment), or, respectively, itching or inflammation subjects would suffer with the onset of dermatitis (prevention).

Furthermore, as previously mentioned, it has been discovered that the ingredients of the combination unexpectedly act in a synergistic manner. Specifically, it has been observed that the cromoglicic acid (or a salt or solvate thereof), preferably disodium cromoglycate (or a solvate thereof), acts synergistically with the rest of constituents of the combination, particularly with the *Croton lechleri* resin and panthenol. In this regard, due to the synergistic effect a lower dosage of the of the cromoglicic acid, or of the *Croton lechleri* resin and panthenol may be required (compared to the sum of the amounts used in monotherapy) to obtain the same or even a better efficacy than with the sum of the efficacies of the respective monotherapies, and the possible toxic side effects that may be observed in monotherapy may be reduced or even prevented. As an alternative, if the dosage of cromoglicic acid, and of the *Croton lechleri* resin and panthenol is the same as when they are provided in monotherapy, an increase in efficacy of the combination that is greater than the sum of the efficacy of monotherapies can be expected.

Therefore, in an embodiment the combinations of the invention or the compositions of the invention are synergistic combinations or compositions.

Likewise, the invention relates to cromoglicic acid (or a salt or solvate thereof), preferably disodium cromoglycate (or a solvate thereof), for use in the treatment of dermatitis, which comprises administering a therapeutically effective amount of cromoglicic acid, or the salt or solvate thereof, in combination, preferably in synergistic combination, with a therapeutically effective amount of *Croton lechleri* resin and panthenol. Likewise, the invention relates to cromoglicic acid (or a salt or solvate thereof), preferably disodium cromoglycate (or a solvate thereof), for use in the treatment of dermatitis, which comprises administering a therapeutically effective amount of cromoglicic acid, or the salt or solvate thereof, in combination, preferably in synergistic combination, with a therapeutically effective amount of *Croton lechleri* resin and panthenol, and additionally with a therapeutically effective amount of aloe vera gel and/or rosehip oil.

In a preferred embodiment, the compositions of the invention are topically applied, preferably on the skin. The topical application can be in affected areas or in unaffected areas (prophylaxis).

In the context of the present invention, the term dermatitis generally includes any inflammatory reaction of the skin.

In a more particular embodiment, dermatitis refers to or is related with atopic dermatitis, contact dermatitis, seborrheic dermatitis, dermatitis herpetiformis, stasis dermatitis, neurodermatitis, traumatic dermatitis, perioral dermatitis, exfoliative dermatitis, dermatitis calorica (dermatitis congelationis or ambustionis), X-ray radiation dermatitis, eczemas, psoriasis, dermatitis derived from vasculitis, Behcet's syndrome, pemphigus, urticaria, urticaria pigmentosa, pyoderma gangrenosum, ulcers, burns, insect stings/bites, herpetic infections, multiple sclerosis (systemic scleroderma), morphea (circumscribed or localized scleroderma), or dermal nodular fibrosis.

In an even more particular embodiment, dermatitis refers to atopic dermatitis, contact dermatitis, seborrheic dermatitis, dermatitis herpetiformis, stasis dermatitis, neurodermatitis, traumatic dermatitis, eczemas, or psoriasis.

In a preferred embodiment, dermatitis refers to atopic dermatitis or contact dermatitis. Even more preferably, dermatitis is atopic dermatitis.

In an embodiment, dermatitis is chronic. In another embodiment, dermatitis is acute.

In the context of the invention, the treatment of dermatitis is understood as being contemplated both in humans and in animals (veterinary use). In a particular embodiment, the subject affected with dermatitis is an animal, for example a cat, dog, or horse. In a preferred embodiment, the subject affected with dermatitis is a human.

In the context of the present invention, the amounts of the combinations or the compositions of the invention that are used for the treatment or prophylaxis of dermatitis, or for preparing a medicinal product intended for the treatment or prophylaxis of dermatitis, are understood to mean therapeutically effective amounts. Therapeutically effective amount is understood to mean that amount which gives rise to an improvement or prevention that can be detected in the patient physiology, i.e., in the skin condition of the patient to whom the combination or composition of the invention is or will be administered.

The amounts to be administered of the combinations of the invention, or the compositions comprising a combination according to the present invention, will vary depending on a number of factors. For example, the dose will vary depending on the specific dermatitis to be treated, particularly it will vary depending on the seriousness or extent of the affected area. A healthcare expert knows to consider the different factors involved in order to determine the specific dose suitable for a given patient.

In a particular embodiment, the compositions comprising a combination according to the present invention are placed in containers, for example packages, which deliver a predetermined amount of composition per pump. Preferably, the container delivers between 1.0 and 0.1 mL, more specifically between 0.7 and 0.2 mL, even more specifically between 0.45 and 0.55 mL per pump. Preferably, the contained composition is in the form of a cream.

The administration regimen of the combinations of the invention, or the compositions comprising a combination according to the present invention, will likewise vary depending on the mentioned different factors. The healthcare expert knows to determine an administration regimen suitable for a given patient.

In a particular embodiment, the combinations of the invention, or the compositions comprising a combination according to the present invention, are administered for the time needed for dermatitis to disappear or for it not to be detectable. In a more specific embodiment, they are administered for one or two months. In another more specific embodiment, they are administered for one, two, or three weeks. In another more specific embodiment, they are administered for 1-6 days, preferably for 6-4 days, more preferably for 5 days.

In a particular embodiment, the combinations of the invention, or the compositions comprising a combination according to the present invention, are administered once, twice, or three times a day; once, twice, or three times every two days; or once, twice, or three times a week. Preferably, they are administered twice a day.

In an even more particular embodiment, they are administered twice a day during any of the aforementioned periods, preferably for one or two months.

In another even more particular embodiment, they are administered three times a day for 1-6 days, preferably for five days.

The administration regimen does not have to be the same throughout the entire treatment, with the understanding that there may be different administration frequencies, for example an initially more frequent administration and a later, less frequent administration.

According to the present invention, the constituents of the combination of the invention
a) cromoglicic acid (or a salt or solvate thereof), preferably disodium cromoglycate (or a solvate thereof);
b) *Croton lechleri* resin;
c) panthenol;
   and, where appropriate,
d) aloe vera gel; and/or rosehip oil,
can be provided in the same pharmaceutical composition, as described above, or in separate compositions for administration at the same time or at different times. If the mentioned constituents are administered at different times, they should be administered close enough together in time, for example they should be administered on the same day, or in a period of two days, in order to assure that the therapeutic efficacy, and particularly, the synergistic response are maintained. Where the constituents are administered in separate compositions, these compositions are prepared and are of the same type as has been described above.

On the other hand, the combinations of the invention, or the compositions comprising a combination according to the present invention, can be used cosmetically for treating dermatitis or reducing the effects derived from dermatitis, for example they can be used as skin regenerators, moisturizers, or antioxidants. In an embodiment, the cosmetic use is performed once inflammation has gone down (the patient no longer suffers inflammation) but the effects thereof continue (for example, wounds). The cosmetic (not therapeutic) use comprises applying on the skin a cosmetically effective amount of the composition or combination of the invention. In the context of the invention, these cosmetic uses are understood to be contemplated both in humans and in animals. In a particular embodiment, the subject is an animal, for example a cat, dog, or horse. In a preferred embodiment, the subject is a human.

### Examples

### Example 1: Preparation of 5% disodium cromoglycate (5% DSCG) composition

The indicated percentages correspond with the weight percentage of each ingredient in the obtained final composition.

In a reactor equipped with previously sterilized stirring equipment, water (q.s.f.), disodium EDTA (0.2%), propylene glycol (5%), glycerin (3%), and D-panthenol (2%) are added, and they are all mixed and heated at 75°C-80°C. In another separate conditioned vessel, Steareth-2 (3.5%), stearyl alcohol (3.5%), Steareth-21 (3%), cetearyl ethylhexanoate (5.7%), paraffin oil (3%), beeswax (0.5%), dimethicone (0.8%), phenoxyethanol (0.67%), stearoxy dimethicone (0.5%), cocoglycerides (0.5%), isopropyl myristate (0.3%), stearic acid (0.2%), and ethylhexylglycerin (0.075%) are added, and they are all mixed and heated at 75°C-80°C. Then an emulsion is prepared from the lipophilic mass and hydrophilic components with suitable stirring. Finally, the obtained emulsion is cooled to 40°C (+/-2°C) .

At the same time as performing the method of emulsion, in a different but likewise conditioned vessel, water (10%) is heated at a temperature of 40°C (+/- 2°C), and disodium cromoglycate (5%) is then slowly added, preventing the formation of lumps, until achieving complete dissolution thereof. A gel is formed.

The aqueous solution of disodium cromoglycate is subsequently added, under stirring, to the emulsion which has been prepared at the same time. The mixture is homogenized. During this addition and homogenization phase, the temperature of 40°C (+/- 2°C) is maintained.

Subsequently, *Croton lechleri* resin (0.045%), propanediol (1.48%), aloe vera gel (10:1 concentrate) (3%), rosehip oil (0.5%), and imidazolidinyl urea (0.25%) are added to the homogenized mixture one by one and in the indicated order. Subsequently, the obtained mixture is homogenized to obtain the composition of the invention. During this addition and homogenization phase, the temperature of 40°C (+/- 2°C) is also maintained. Finally, the pH was adjusted by the addition of NaOH (0.5%) .

### Example 2: In vivo study of the treatment of dermatitis

The following formulations were tested in a mouse model of dermatitis:

| Group of mice | Applied formulation |
|---|---|
| C | none |
| DC | none |
| D | 5% DSCG |
| D | 2% DSCG |
| D | No DSCG |
| D | 2% DSCG (no CL/P) |

| | |
|---|---|
| wherein C = control (mice without dermatitis); DC = dermatitis control (sensitized mice not receiving treatment); D = dermatitis (sensitized mice receiving treatment). | |

The 5% DSCG formulation is the composition prepared according to Example 1.

The 2% DSCG formulation is a composition prepared following the method of preparation of Example 1, but in which an amount of disodium cromoglycate representing only 2% by weight with respect to the total weight of the composition was added.

The No DSCG formulation is a comparative composition prepared following the method of preparation of Example 1 but in which the step of adding disodium cromoglycate was omitted, such that the composition does not comprise disodium cromoglycate.

The 2% DSCG (no CL/P) formulation is a composition prepared following the method of preparation of Example 1 but in which the step of adding *Croton lechleri* resin and panthenol was omitted, and in which an amount of disodium cromoglycate representing only 2% by weight with respect to the total weight of the composition was added.

### Animal model of dermatitis

The following sensitization protocol was followed to induce atopic dermatitis in mice. 2,4-dinitrofluorobenzene (DNFB) was applied on the outer surface of both the ears of BALB/c strain mice every 2 days. After 19 days, the application of DNFB was stopped. It was observed that ear thickness increased during the sensitization phase from an initial 0.20 mm to about 0.48 mm (an increase of 0.28 mm or 100%) in 18.

### Treatment

Once the application of DNFB was stopped, the application of the different formulations (creams) was started. Two groups of mice were used as controls: a first group "C" which was neither sensitized nor treated with any formulation, and a second group "CD", used as the dermatitis control, which was sensitized but not treated with a formulation.

The tested formulations (5% DSCG, 2% DSCG, No DSCG, 2% DSCG (no CL/P)) were applied on the ears of a respective group of mice for 7 days, and the ears were collected.

All the groups of mice consisted of 6 mice (12 ears).

### Results

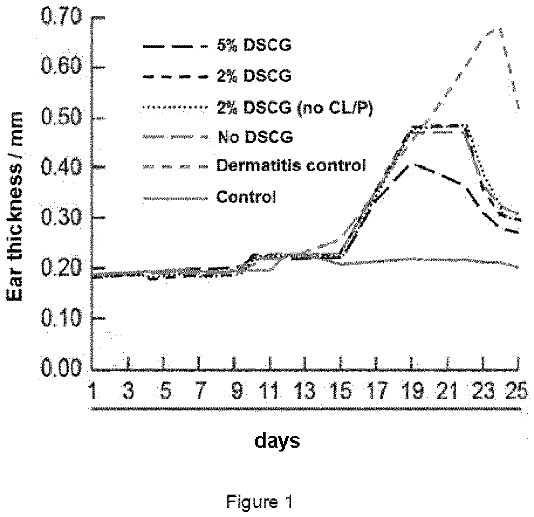

As was to be expected, no significant variation in ear thickness was observed in group C (Figure 1). In the DC group, after stopping the application of DNFB, ear thickness continued to increase for 5 days, reaching 0.68 mm, after which thickness started to decrease. In the other cases, in which one of the tested formulations was applied after stopping the application of DNFB, two different behaviors were observed. In the groups treated with the 2% DSCG, No DSCG, and 2% DSCG (no CL/P) formulations, ear thickness stopped increasing and remained relatively constant for 3 days, with a decrease in ear thickness being observed in the next 3 days until reaching an ear thickness of about 0.30 mm (a reduction of 0.18 mm or 64%) after 6 days. In the group treated with the 5% DSCG formulation, a surprising synergistic effect was observed which involved the reduction of ear thickness from the time that the formulation was applied, and a quicker and more prominent reduction, with a thickness of 0.27 mm (a reduction of 0.21 mm or 75%) being achieved after 6 days.

### Example 3: In vivo study II of the treatment of dermatitis Animal model of dermatitis

The following sensitization protocol was followed to induce atopic dermatitis in mice. 2,4-dinitrofluorobenzene (DNFB) was applied on the outer surface of both the ears of BALB/c strain mice. The application of DNFB was stopped when ear thickness reached 0.55 mm.

### Treatment

Once the application of DNFB was stopped, the application of the 5% DSCG and 2% DSCG formulations (creams) was started. Two groups of mice were used as controls: a first group "C" which was neither sensitized nor treated with any formulation, and a second group "CD", used as the dermatitis control, which was sensitized but not treated with a formulation.

The tested formulations (5% DSCG, 2% DSCG) were administered, respectively, to two groups of mice for 9 days, and the ears were collected.

All the groups of mice consisted of 4 mice (8 ears).

### Results

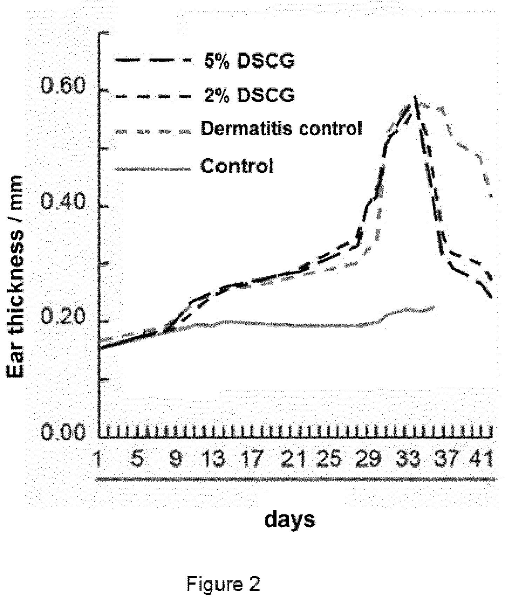

As was to be expected, no significant variation in ear thickness was observed in group C (Figure 2). In the groups treated with formulations, after three days of treatment with the formulations ear thickness decreased rapidly, whereas in the DC group ear thickness remained around 0.57 mm. After 8 days of treatment, ear thickness in the 5% DSCG group decreased to 0.25 mm; in the 2% DSCG group it decreased to 0.28 mm; in the DC group it decreased to 0.42 mm.

### Example 4: In vivo study III of the treatment of dermatitis

A protocol similar to the protocol of Example 2 was followed, in which the sensitization phase lasted for 7 days (first week) and the compositions were applied during the second week. Again, similar results were observed, particularly in relation to the synergistic activity of the 5% DSCG composition on dermatitis. The results observed for the different tested compositions are shown numerically below:

| Composition | Decrease in ear thickness |
|---|---|
| No DSCG | 7 mm |
| 2% DSCG (no CL/P) | 4 mm |
| 5% DSCG | 20 mm |

The theoretical additive activity for the 5% DSCG formulation was a reduction of 17 mm (7 mm that could be attributed to *Croton lechleri* resin and panthenol, and 10 mm that could be attributed to DSCG [2.5 x 4 mm]). However, the reduction was unexpectedly observed to be greater, specifically 20 mm, whereby showing the synergistic effect taking place when using the combinations of the present invention.

### Example 5: In vivo study of the prevention of dermatitis

The assay of Example 2 was followed with some modifications. Specifically, the formulations were applied at the same time as the application of 2,4-dinitrofluorobenzene (DNFB). It was observed that the 5% DSCG and 2% DSCG formulations were able to prevent the thickening of the ear compared with the dermatitis control group. Furthermore, it was again observed that the action of the 5% DSCG formulation acts synergistically. It was observed that the 5% DSCG formulation has a high capacity to prevent pruritus and inflammation.

### Example 6: Study of PAR2, TRPV4, and TNF-alpha levels

The tissue of the ears obtained after the assay of Example 4 was ground up and homogenized (glass on glass) at 4°C using RIPA buffer (Thermo-Fisher Scientific) formed by a protease and phosphatase inhibitor mixture (Roche Diagnostics, Indianapolis, IN). The samples were sonicated and centrifuged for 10 minutes a 14,000 g. The protein concentration was determined with a Bradford assay (Bio-Rad, Hercules, CA). The supernatant was used for sodium dodecylsulfate polyacrylamide gel electrophoresis (SDS-PAGE), and the pellet was stored at -20°C. Equal amounts of boiled protein (20 µg per lane) were diluted with SDS buffer, loaded in pre-molded BioRad Criterion gels (4-12%) for SDS-PAGE (BioRad, Hercules, CA), and subjected to electrophoresis at 125 V for 1.5 hours on ice. The proteins were studied in polyvinylidene difluoride (PVDF) membranes using the BioRad Criterion TransBlot system. Blotting was carried out in a transblot turbo system (BioRad) following the manufacturer's instructions. The retention of gel was evaluated by means of Coomassie blue staining (Pierce, Rockford, IL). The non-specific binding was blocked for 1 hour at room temperature with SuperBlock blocking solution (Thermo-Fisher Scientific). The membranes were incubated overnight at 4°C with PAR2-, TRPV4-, and TNF-α-specific primary antibodies in TTBS. The bound primary antibody was detected by means of a HRP-conjugated secondary antibody at 55, 98, and 17 kDa, respectively. The proteins bands were viewed with a digital Fujifilm LAS-3000 scanner following the manufacturer's instructions and were quantified using ImageQuant TL software (GE Healthcare). The membranes were separated for 30 minutes, tested again with GAPDH (38 kDa, monoclonal mouse IgG) as the loading control (EMD Millipore), and detected with anti-mouse IgG HRP (Santa Cruz).

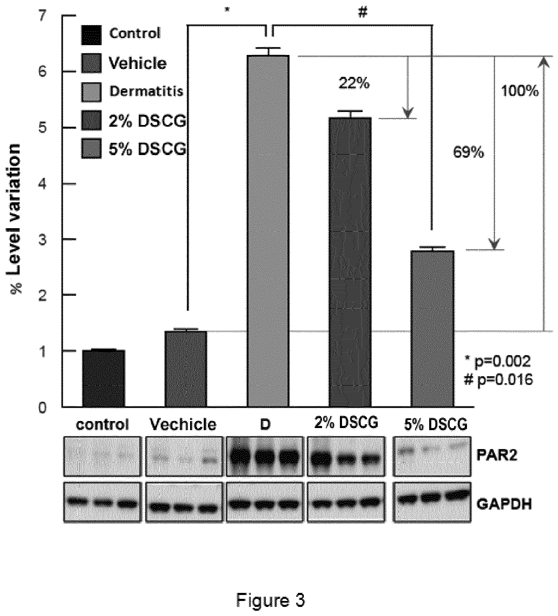

The results are shown in Figures 3 (PAR2), 4 (TRPV4), and 5 (TNF-alpha). A clear reduction of the levels of these three markers was observed in the samples derived from the ears that were treated with the combinations of the invention, and more particularly with the 5% DSCG composition for which, once again, a synergistic effect is shown by means of the decrease in PAR2 levels by more than three-fold (from 22% to 69%), while DGSC only increases 2.5-fold (from 2% to 5%).

## Claims

1. A combination comprising:
a) cromoglicic acid or a salt or solvate thereof;
b) *Croton lechleri* resin; and
c) Panthenol
wherein the proportion by weight between the weight of the cromoglicic acid or the salt or solvate thereof, and the weight of panthenol, is 2.5:2.000 or greater.

2. The combination according to claim 1, wherein the cromoglicic acid is disodium cromoglycate or a solvate thereof.

3. The combination according to any of the preceding claims, wherein the proportion by weight between the cromoglicic acid or the salt or solvate thereof, and panthenol, is 4.5:2.000 or greater.

4. The combination according to any of the preceding claims, additionally comprising d) aloe vera gel and/or rosehip oil.

5. A pharmaceutical composition comprising:
a) a combination as defined in any of claims 1 to 4; and
b) a pharmaceutically acceptable vehicle.

6. The pharmaceutical composition according to claim 5, wherein the combination comprises between 2.5% and 10% by weight of cromoglicic acid with respect to the total weight of the pharmaceutical composition.

7. The pharmaceutical composition according to claim 6, wherein the combination comprises between 4.5% and 5.5% by weight of cromoglicic acid with respect to the total weight of the pharmaceutical composition.

8. The pharmaceutical composition according to any of claims 5 to 7, additionally comprising at least one active ingredient which is selected from corticosteroids, antihistamines, antiallergic agents, and antibacterial agents.

9. The pharmaceutical composition according to any of claims 5 to 8, wherein the pharmaceutical composition is an oil-in-water type emulsion.

10. The pharmaceutical composition according to any of claims 5 to 9, wherein the pharmaceutical composition is a cream, lotion, or milk.

11. The combination according to any of claims 1 to 4 or the composition according to any of claims 5 to 10 for use as a medicament.

12. The combination or composition for use according to claim 11, for use in the treatment or prophylaxis of atopic dermatitis.

13. The combination or composition for use according to claim 11 for use in the treatment or prophylaxis of the itching and/or inflammation associated with atopic dermatitis.

14. Non-therapeutic use of the combination according to any of claims 1 to 4 or the composition according to any of claims 5 to 10 for treating cosmetic effects derived from atopic dermatitis.

## Patentansprüche

1. Kombination, umfassend:
a) Cromoglicinsäure oder ein Salz oder Solvat davon;
b) Harz von *Croton lechleri;* und
c) Panthenol
wobei das Gewichtsverhältnis zwischen dem Gewicht der Cromoglicinsäure oder des Salzes oder Solvats davon und dem Gewicht des Panthenols 2,5:2,000 oder mehr beträgt.

2. Die Kombination nach Anspruch 1, wobei die Cromoglicinsäure Dinatriumcromoglycat oder ein Solvat davon ist.

3. Die Kombination nach einem der vorhergehenden Ansprüche, wobei das Gewichtsverhältnis zwischen der Cromoglicinsäure oder des Salzes oder Solvats davon und Panthenol 4,5:2,000 oder mehr beträgt.

4. Die Kombination nach einem der vorhergehenden Ansprüche, zusätzlich umfassend d) Aloe Vera-Gel und/oder Hagebuttenöl.

5. Pharmazeutische Zusammensetzung, umfassend:
a) eine Kombination wie in einem der Ansprüche 1 bis 4 definiert; und
b) ein pharmazeutisch akzeptables Vehikel.

6. Die pharmazeutische Zusammensetzung nach Anspruch 5, wobei die Kombination zwischen 2,5 und 10 Gew.-% Cromoglicinsäure, bezogen auf das Gesamtgewicht der pharmazeutischen Zusammensetzung, umfasst.

7. Die pharmazeutische Zusammensetzung nach Anspruch 6, wobei die Kombination zwischen 4,5 und 5,5 Gew.-% Cromoglicinsäure, bezogen auf das Gesamtgewicht der pharmazeutischen Zusammensetzung, umfasst.

8. Die pharmazeutische Zusammensetzung nach einem der Ansprüche 5 bis 7, zusätzlich umfassend zumindest einen aktiven Inhaltsstoff, der ausgewählt ist aus Kortikosteroiden, Antihistaminika, antiallergischen Mitteln und antibakteriellen Mitteln.

9. Die pharmazeutische Zusammensetzung nach einem der Ansprüche 5 bis 8, wobei die pharmazeutische Zusammensetzung eine Emulsion vom Typ Öl-in-Wasser ist.

10. Die pharmazeutische Zusammensetzung nach einem der Ansprüche 5 bis 9, wobei die pharmazeutische Zusammensetzung eine Creme, Lotion oder Milch ist.

11. Die Kombination nach einem der Ansprüche 1 bis 4 oder die Zusammensetzung nach einem der Ansprüche 5 bis 10 zur Verwendung als Medikament.

12. Die Kombination oder Zusammensetzung zur Verwendung gemäß Anspruch 11 zur Verwendung bei der Behandlung oder Prophylaxe von atopischer Dermatitis.

13. Die Kombination oder Zusammensetzung zur Verwendung gemäß Anspruch 11 zur Verwendung bei der Behandlung oder Prophylaxe des Juckens und/oder der Entzündung, die mit der atopischen Dermatitis verbunden sind.

14. Nichttherapeutische Verwendung der Kombination nach einem der Ansprüche 1 bis 4 oder der Zusammensetzung nach einem der Ansprüche 5 bis 10 zur Behandlung kosmetischer Effekte, die von atopischer Dermatitis herrühren.

## Revendications

1. Une combinaison comprenant :
a) de l'acide cromoglicique ou un sel ou solvate de celui-ci ;
b) une résine *Croton lechleri* ; et
c) du Panthénol
la proportion en poids entre le poids de l'acide cromoglicique ou de son sel ou solvate et le poids du panthénol est de 2,5:2,000 ou plus.

2. La combinaison selon la revendication 1, dans laquelle l'acide cromoglicique est le cromoglycate disodique ou un solvate de celui-ci.

3. La combinaison selon l'une quelconque des revendications précédentes, dans laquelle la proportion en poids entre l'acide cromoglicique ou son sel ou solvate et le panthénol est de 4,5:2,000 ou plus.

4. La combinaison selon l'une quelconque des revendications précédentes, comprenant en outre d) du gel d'aloe vera et/ou de l'huile de rose musquée.

5. Une composition pharmaceutique comprenant :
a) une combinaison telle que définie dans l'une quelconque des revendications 1 à 4 ; et
b) un véhicule pharmaceutiquement acceptable.

6. La composition pharmaceutique selon la revendication 5, dans laquelle la combinaison comprend entre 2,5% et 10% en poids d'acide cromoglicique par rapport au poids total de la composition pharmaceutique.

7. La composition pharmaceutique selon la revendication 6, dans laquelle la combinaison comprend entre 4,5% et 5,5% en poids d'acide cromoglicique par rapport au poids total de la composition pharmaceutique.

8. La composition pharmaceutique selon l'une quelconque des revendications 5 à 7, comprenant en outre au moins un principe actif choisi parmi les corticostéroïdes, les antihistaminiques, les agents antiallergiques et les agents antibactériens.

9. La composition pharmaceutique selon l'une quelconque des revendications 5 à 8, dans laquelle la composition pharmaceutique est une émulsion de type huile dans l'eau.

10. La composition pharmaceutique selon l'une quelconque des revendications 5 à 9, dans laquelle la composition pharmaceutique est une crème, une lotion ou un lait.

11. La combinaison selon l'une quelconque des revendications 1 à 4 ou composition selon l'une quelconque des revendications 5 à 10 pour une utilisation en tant que médicament.

12. La combinaison ou la composition à utiliser selon la revendication 11, pour une utilisation dans le traitement ou la prophylaxie de la dermatite atopique.

13. La combinaison ou la composition à utiliser selon la revendication 11, pour une utilisation dans le traitement ou la prophylaxie des démangeaisons et/ou de l'inflammation associées à la dermatite atopique.

14. Utilisation non-thérapeutique de la combinaison selon l'une des revendications 1 à 4 ou de la composition selon l'une des revendications 5 à 10 pour traiter les effets cosmétiques dérivés de la dermatite atopique.
